# EUROPEAN PATENT APPLICATION

(11) **EP 3 096 141 A1**
(43) Date of publication of application: **23.11.2016**
(21) Application number: 15737047.9
(22) Date of filing: 14.01.2015
(51) Int. Cl.: G01N 33/574, G01N 33/53, G01N 33/48

(54) **METHOD FOR SCREENING CANCER PREVENTION AGENT OR ANTICANCER AGENT USING MORPHOLOGICAL CHARACTERISTICS OF LUTERIAL**

(30) Priority: 14.01.2014 KR 20140004527
(71) Applicant: Choi, Won Cheol, Incheon 402-765 (KR); Kwon, Young Ah, Seoul 135-787 (KR); Choi, Suk Hoon, Seoul 135-787 (KR); Choi, Chang Hoon, Seoul 135-787 (KR)
(72) Inventor: CHOI, Won Cheol, Incheon 22143 (KR); KWON, Young Ah, Seoul 06002 (KR); CHOI, Suk Hoon, Seoul 06002 (KR); CHOI, Chang Hoon, Seoul 06002 (KR); KWON, Sung Pil, Seoul 138-747 (KR); JUN, Hyun Jung, Somerville, Massachusetts 02143 (US)
(74) Representative: Ladendorf, Oliver
(86) International application number: PCT/KR2015/000405
(87) International publication number: WO 2015/108342

(57) **Abstract**

The present invention relates to a method for screening an anticancer agent or a cancer preventive agent, comprising the steps of: (a) isolating mutant luterial or normal luterial from a body fluid extracted from a patient or a normal person; (b) treating the isolated mutant luterial with anticancer agent candidates or cancer preventive agent candidates; and (c) either selecting, as the anticancer agent, a candidate that reduces the size or changes the shape or increases the mobility of the mutant luterial, compared to the control mutant luterial upon treatment with the candidate, or selecting, as the cancer preventive agent, a candidate that suppresses the increase in size, minimizes the change in shape or maintains the mobility of luterial, compared to the control luterial, upon treatment with the candidate.

## Description

### TECHNICAL FIELD

The present invention relates to a method of screening an anticancer agent based on the morphological characteristics of luterial, and more particularly, to a method for screening and selecting an anticancer agent or a cancer preventive candidate based on its ability to induce a change in the size, shape or mobility of mutant luterial or inhibit a change in the size, shape or mobility of luterial, compared to control mutant luterial or luterial before treatment with such agent or candidate.

### BACKGROUND ART

Cancer is one of the major diseases that threaten human health, and is a disease in which cells proliferate in an unrestricted and uncontrolled manner through a series of mutation processes. Various biochemical mechanisms associated with cancer have been identified, and improved methods for detection and mass screening of cancer have been developed in the past decade. Despite such improvement, however, a method for fundamentally curing cancer is yet to be proposed. In Particular, the treatment for terminal cancer has been very limited to date.

As a variety of anticancer agents have been continuously developed and clinically applied, the anticancer therapeutic effects thereof have also increased. However, the response rate to the treatment of a variety of cancers is still insufficient, and for this reason, the effect of alleviating symptoms or extending the life span of cancer patients is low, even with a considerable number of cancer patients being exposed to anticancer agents with a very strong cytotoxicity. To improve the current situation, a search for better anticancer agents and standard methods for effective treatment of cancer is required.

Under such circumstances, efforts have been made to develop anticancer agents by identifying various biological molecules associated with cancer and screening drugs that are able to target such molecules. Anticancer agent screening is a process in which it evaluates the therapeutic activity and cytotoxicity of anticancer agent candidates (such as synthetic compounds or natural substances).

Specifically, anticancer agent screening may refer to a series of processes in which an effect on the death or inhibition of proliferation of cancer cells in culture is compared between samples treated with various anticancer agents and an untreated sample. It thereby finds the most ideal anticancer agent or selects out an anticancer agent believed to have no effect, and also measures the extent of death or inhibition of proliferation of cancer cells exposed to anticancer agents.

As a conventional *in vivo* screening method, a subrenal capsule assay has been used, : extracting tumor tissue from a patient; cutting the extracted tumor tissue to a small size; transplanting the cut tissue into the subrenal capsule of a mouse; treating the mouse with an anticancer agent; and measuring a change in the size of the transplanted tumor. However, this method has a disadvantage of inefficiency for screening a large number of anticancer agents because of a long time required to screen for the dose- and injection frequency dependent effects of the anticancer agent.

In addition, *in vitro* screening methods comprise: isolating cells from cancer tissue; treating the isolated cells with an anticancer agent; culturing the treated cells; and determining whether the cells would be dead or whether the proliferation of the cells would be inhibited.

In order to screen a suitable anticancer agent based on the experimental results obtained from the above methods, a process of analyzing a response with an anticancer agent and the experimental results is required. Specifically, comparison with previously established *in vitro* data, including the examination of cell death rate (or proliferation inhibitory rate), IC₅₀, chemical sensitivity index, the relative distribution of reactivity in an anticancer agent reactivity database, etc., may be performed.

Through this process, an anticancer agent believed to have an effect can be discriminated from an anticancer agent believed to have no effect, and accuracy can be increased by repeating this process.

In this context, the present inventors found that a disease can be diagnosed and predicted by observing the characteristics of a micro-substance present in a body fluid extracted from a patient. The content of this finding was filed for a patent on January 14, 2014 (PCT/KR2014/00393). The present inventors named the micro-substance a "luterial".

In addition, the present inventors developed a method capable of effectively isolating the micro-substance luterial present in a body fluid extracted from a patient or a normal person and characterized the isolated luterial. The content of this development and characterization was filed for a patent on May 9, 2014 (PCT/KR2014/004197).

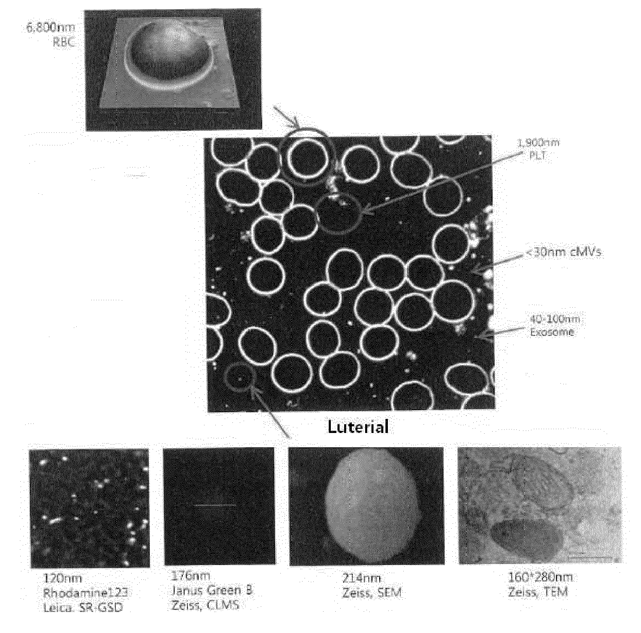

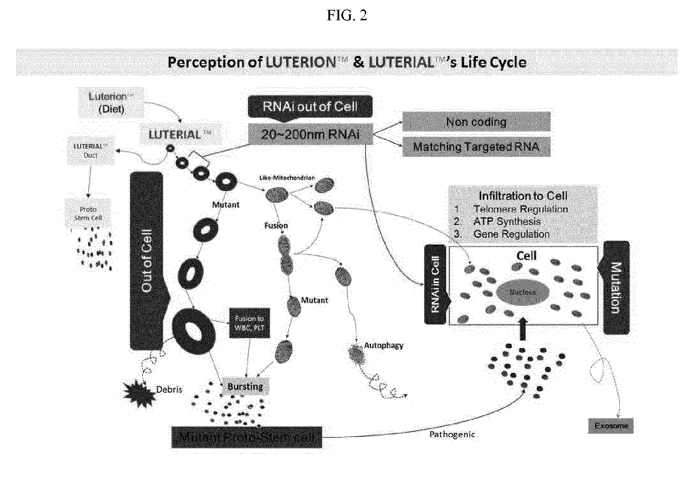

Luterial is discriminated from exosomes or microvesicles in that it includes both DNA and RNA and is adherent and mobile (FIG. 1). In the case of mammals (including humans), **luterial** named by the present inventors is present in blood, saliva, lymphatic ducts, semen, vaginal fluids, mother's milk (particularly colostrums), umbilical cord blood, brain cells, spinal cords, and marrow. In addition, a luterial-like substance present in plants is referred to as "**luterion**", and the origin of luterial that is found in body fluids such as blood is presumed to be from stem cells or red blood cells or from the intake of plant-derived luterion (FIG. 2).

Luterial has the following characteristics: (1) it is a cell or cell-like structure having fusion characteristics corresponding to those of an intermediate stage between a prokaryote and an eukaryote; (2) it is present in body fluids, including blood, semen, intestinal juices, saliva, cellular fluids, etc.; (3) it shows a positive staining reaction with Janus green B, Acridine Orange and Rhodamine 123 in a fluorescence test; (4) in an optimal environment (pH 7.2-7.4), it has the property of expressing genes homologous to beta-proteobacteria and gamma-proteobacteria and has a size of 30-800 nm; (5) in an acidic environment, it expresses not only genes homologous to beta-proteobacteria and gamma-proteobacteria, but also eukaryotic genes (particularly Streptophyta genes), and grows to a size of 400-2000 nm or more; (6) it is involved in ATP production under normal conditions; and (7) it is a cell-like structure which differs from mitochondria and completely differs from exosomes (PCT/KR2014/004197).

In the case of mammals (including humans), luterial is present in blood, saliva, lymphatic ducts, semen, vaginal fluids, mother's milk (particularly colostrums), umbilical cord blood, brain cells, blood cells, stem cells, spinal cords, and marrow. In addition, in the case of horned animals, luterial is also present in horns (PCT/KR2014/00393).

Normal luterials have a size of 50-800 nm, and mutant luterials formed by fusion have a size of a few tens of micrometers. The term "luterial" may refer to proto mitochondria containing mRNA, miRNA and DNA. Luterial is unique in that it does not dissolve in digestive fluid and infiltrates into blood (PCT/KR2014/004197).

It is expected that luterial will be associated with not only signal transduction, cell differentiation and cell death, but also the regulation of cell cycling and cell growth. The present inventors have found that luterial is closely associated with the diagnosis of cancer (PCT/KR2014/00393).

The present inventors have found that, using this luterial or its mutant, one is able to screen and identify an agent among candidates, that prevents or treats cancer by promoting the maintenance of normal state of luterial or by inhibiting, reducing or restoring the abnormal state of luterial, thereby completing the present invention.

### DISCLOSURE OF INVENTION

### TECHNICAL PROBLEM

It is an object of the present invention to provide a method of screening an anticancer agent useful for the prevention or treatment of cancer by using mutant luterial, which is found specifically in cancer patients, as a cancer marker or a target for cancer treatment.

### TECHNICAL SOLUTION

To achieve the above object, the present invention provides a method for screening an anticancer agent, comprising the steps of:
(a) isolating mutant luterial from a body fluid extracted from a patient;
(b) treating the isolated mutant luterial with anticancer agent candidates; and
(c) selecting, as the anticancer agent, a candidate that reduces the size, changes the shape or increases the mobility of the mutant luterial, compared to that of a control mutant luterial without treatment with the candidate.

The present invention also provides a method for screening a cancer preventive agent, comprising the steps of:
(a) isolating normal luterial from a body fluid extracted from a normal person;
(b) culturing the isolated normal luterial in the presence of cancer preventive agent candidates; and
(c) selecting, as the cancer preventive agent, a candidate that suppresses the increase in size, minimizes the change in shape or maintains the mobility of luterial, compared to that of a control luterial without treatment with the candidate.

### BRIEF DESCRIPTION OF THE DRAWINGS

FIG. 1 shows the results of observing normal luterial with an optical microscope (SR-GSD or CLSM) or an electron microscope (SEM or TEM).
FIG. 2 schematically shows the life cycle of luterial.
FIG. 3 is a schematic view showing a cancer development mechanism associated with luterial.
FIG. 4 is a dark-field microscope image of flagellum-shaped luterial.
FIG. 5 is a dark-field microscope image of micro-tubular luterial.
FIG. 6 is a dark-field microscope image of mass-shaped luterial.
FIG. 7 is a dark-field microscope image of rod-shaped luterial.
FIG. 8 is a dark-field microscope image of luterial derived from a stage 4 lung cancer patient.

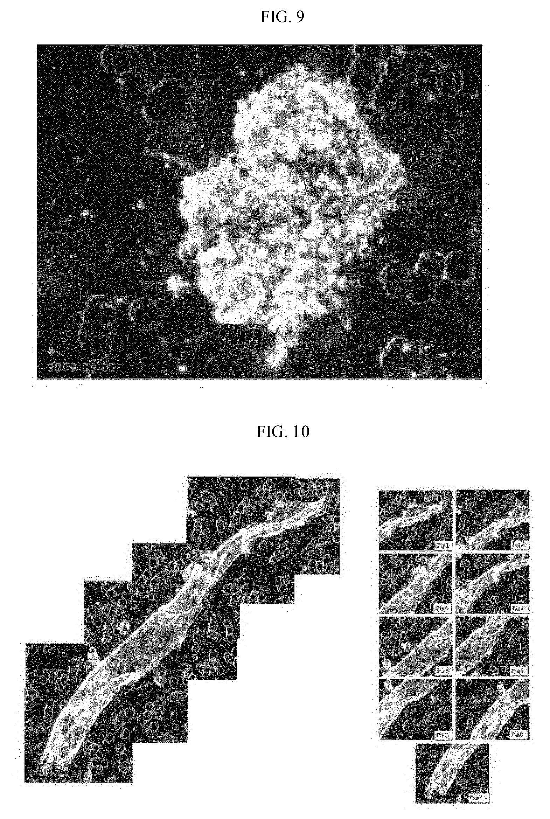

FIG. 9 is a dark-field microscope image of luterial derived from a lung cancer patient.
FIG. 10 is a dark-field microscope image of luterial derived from a stage 3b lung cancer patient.

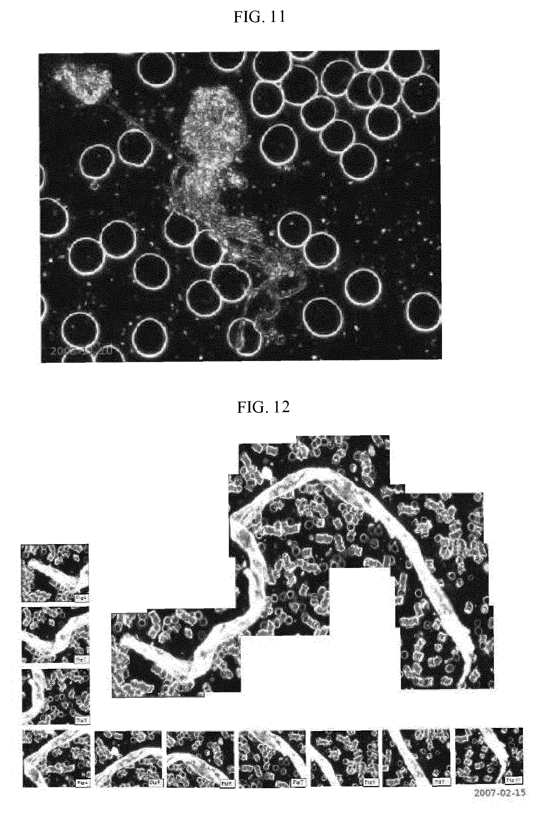

FIG. 11 is a dark-field microscope image of luterial derived from a lung cancer patient.
FIG. 12 is a dark-field microscope image of luterial derived from a patient with non-small cell lung cancer metastasized to the brain.

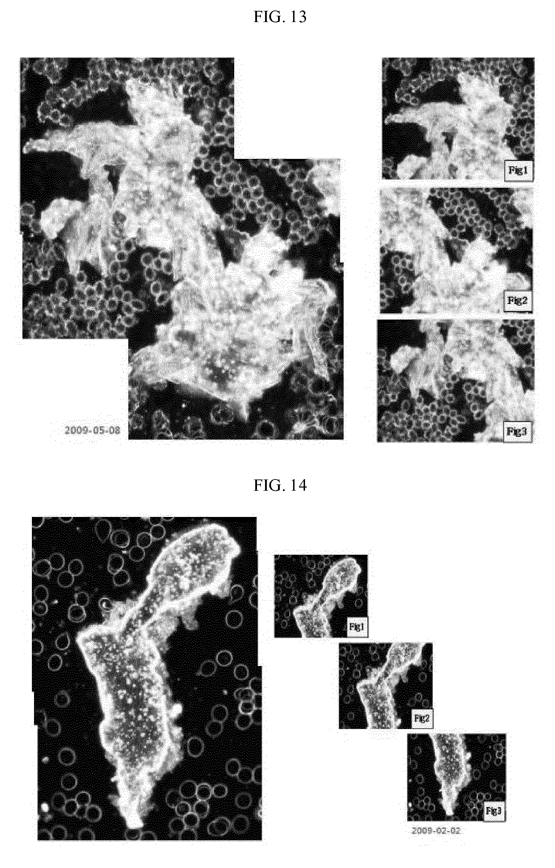

FIG. 13 is a dark-field microscope image of luterial derived from a patient with lung cancer (squamous cell carcinoma) metastasized to supraclavicular lymph nodes and the liver.
FIG. 14 is a dark-field microscope image of luterial derived from a patient with lung cancer metastasized to bone.

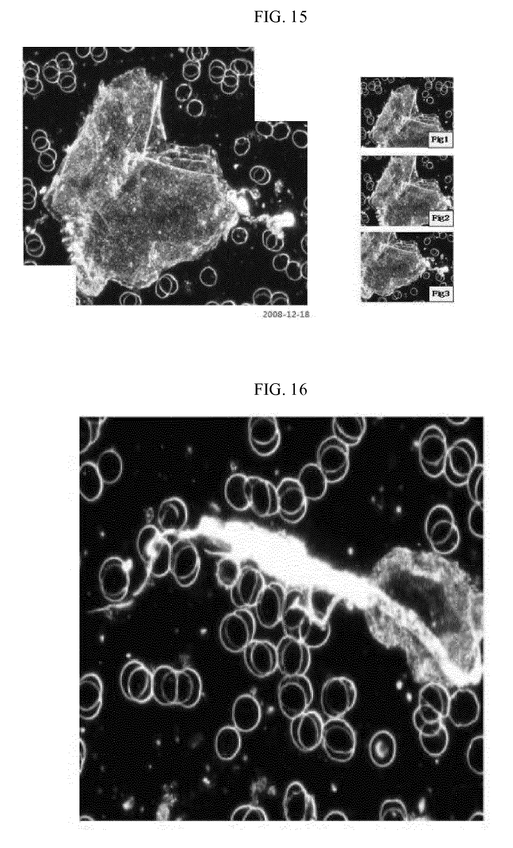

FIG. 15 is a dark-field microscope image of luterial derived from a lung cancer patient.
FIG. 16 is a dark-field microscope image of luterial derived from a lung cancer patient.

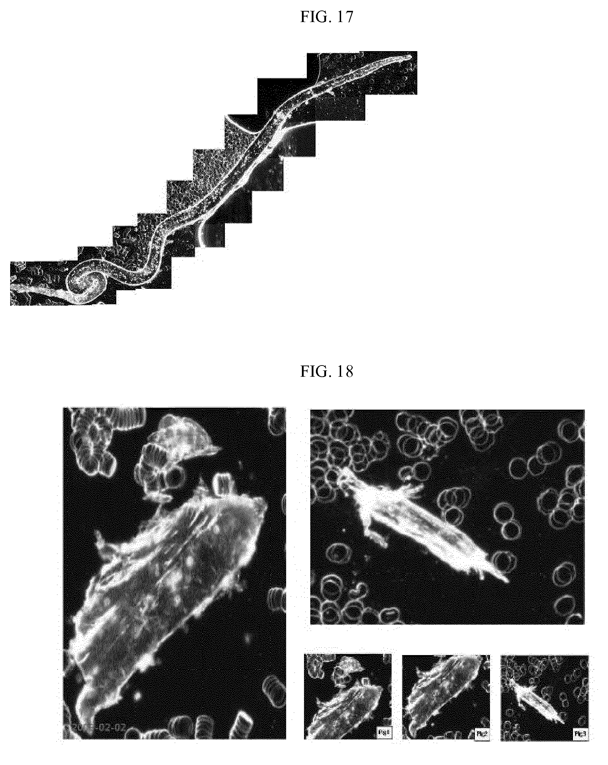

FIG. 17 is a dark-field microscope image of luterial derived from a pancreatic cancer patient.
FIG. 18 is a dark-field microscope image of luterial derived from a patient with pancreatic cancer metastasized to the liver.

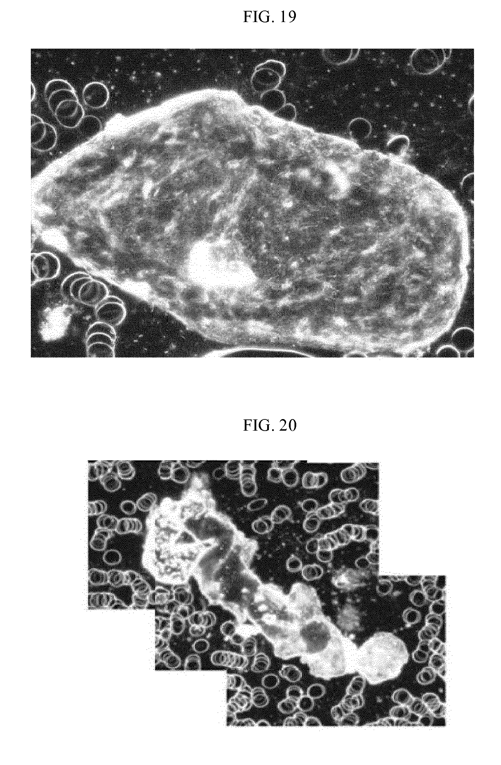

FIG. 19 is a dark-field microscope image of luterial derived from a patient with colorectal cancer metastasized to the uterus.
FIG. 20 is a dark-field microscope image of luterial derived from a patient with colorectal cancer metastasized to the liver and the lungs.

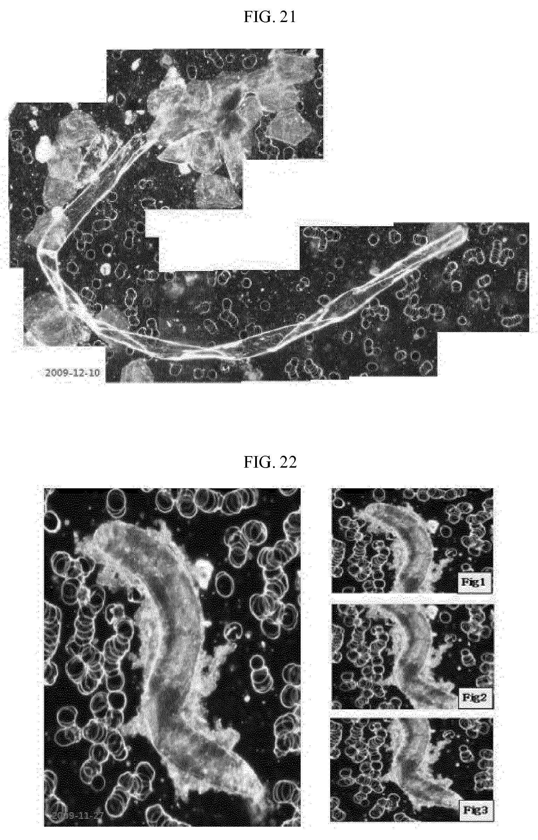

FIG. 21 is a dark-field microscope image of luterial derived from a patient with colorectal cancer metastasized to the liver, the lungs and the brain.
FIG. 22 is a dark-field microscope image of luterial derived from a patient with colorectal cancer metastasized to the liver.

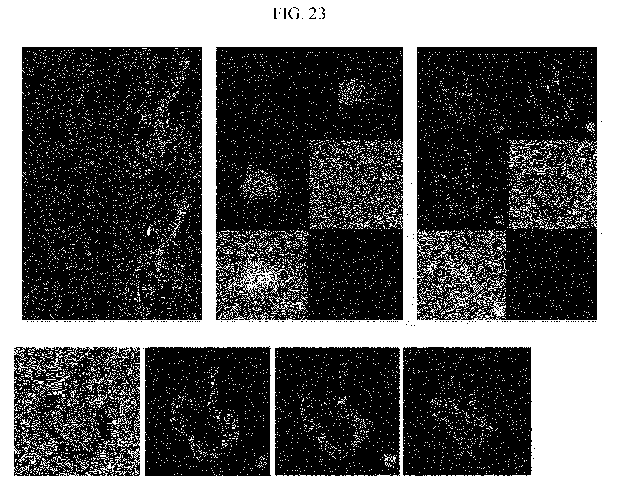

FIG. 23 is a dark-field microscope image of luterial derived from a patient with liver cancer metastasized to the lungs.

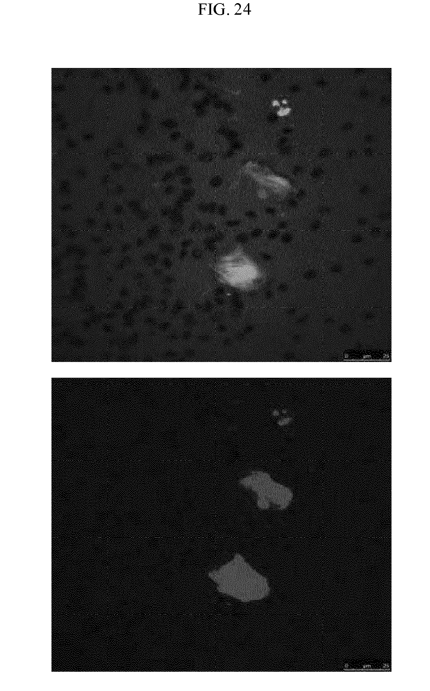

FIG. 24 is a confocal laser scanning microscope image of luterial derived from a patient with angiosarcoma of the liver.

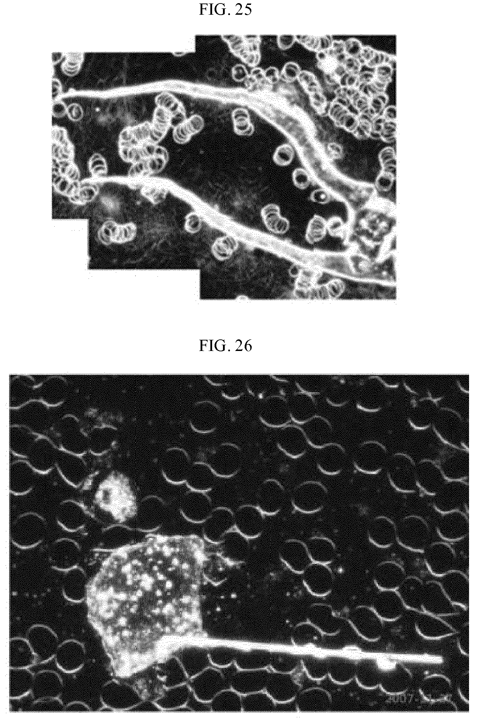

FIG. 25 is a dark-field microscope image of luterial derived from a patient with terminal gallbladder cancer.
FIG. 26 is a dark-field microscope image of luterial derived from a patient with prostate cancer metastasized to bone.

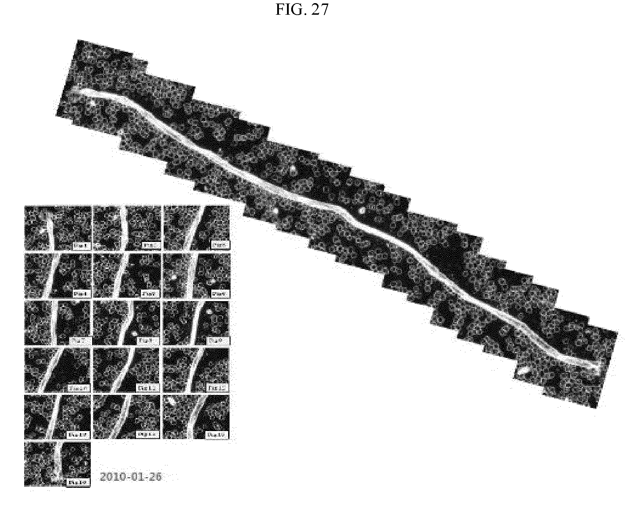

FIG. 27 is a dark-field microscope image of luterial derived from a patient with prostate cancer.

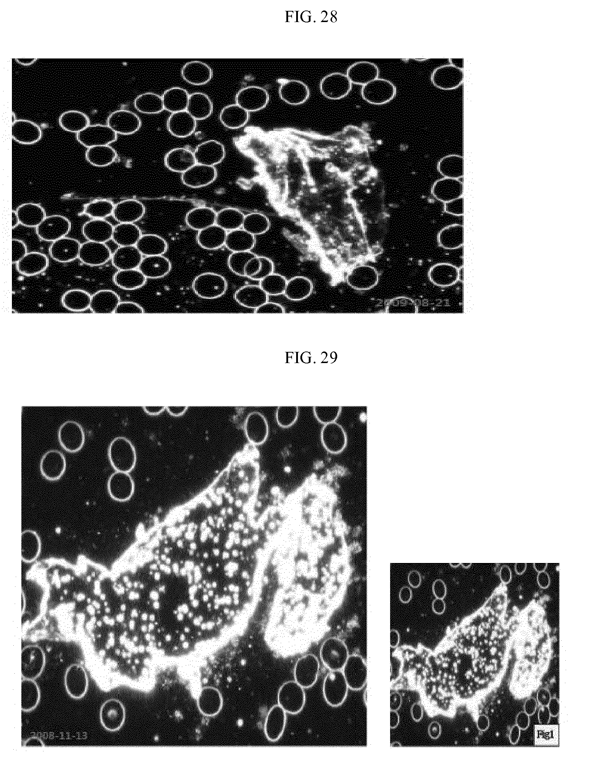

FIG. 28 is a dark-field microscope image of luterial derived from a stage 3 breast cancer patient.
FIG. 29 is a dark-field microscope image of luterial derived from a stage 3b breast cancer patient.

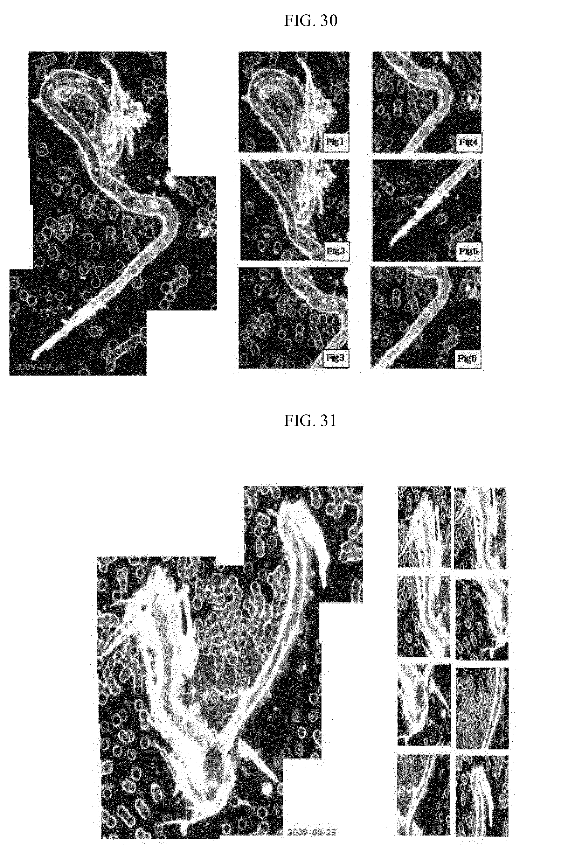

FIG. 30 is a dark-field microscope image of luterial derived from a patient with thyroid papillary carcinoma.
FIG. 31 is a dark-field microscope image of luterial derived from a renal cancer patient.

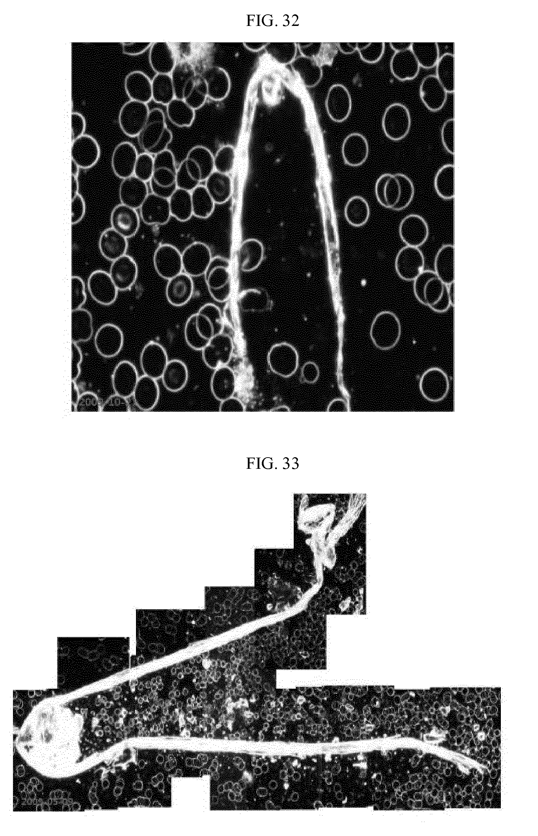

FIG. 32 is a dark-field microscope image of luterial derived from a gastric cancer patient.
FIG. 33 is a dark-field microscope image of luterial derived from a gastric cancer patient.

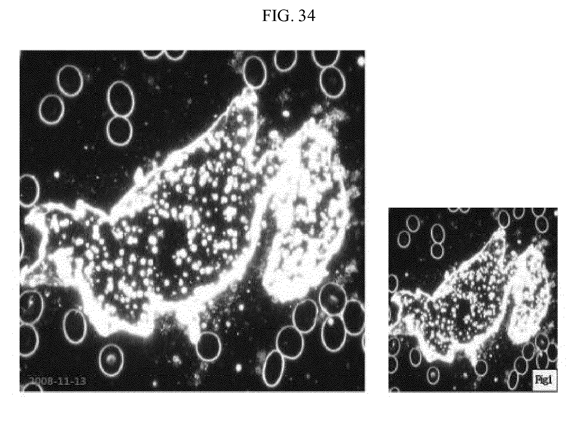

FIG. 34 is a dark-field microscope image of luterial derived from a stage 3b breast cancer patient.
FIG. 35 is a dark-field microscope image of luterial (stage 1) from the blood of a normal person.
FIG. 36 shows the change in size or shape or the restoration of mobility of mutant luterial when it is treated with luterion isolated from *Rhus verniciflua stokes (candidate).*
   (a): before treatment with the candidate; (b): 30 min treatment with the candidate; and (c) : 1 hour treatment with the candidate.
FIG. 37 shows the change in size or shape or the restoration of mobility of mutant luterial when it is treated with luterion isolated from *Forsythiae fructus* (candidate). (a): before treatment with the candidate; (b): 30 min treatment with the candidate; and (c): 1 hour treatment with the candidate.
FIG. 38 shows the change in size or shape or the restoration of mobility of mutant luterial when it is treated with luterion isolated from *Poria cocas (candidate).* (a): before treatment with the candidate; (b): 30 min treatment with the candidate; and (c): 1 hour treatment with the candidate.
FIG. 39 shows the change in size or shape or the restoration of mobility of mutant luterial when it is treated with luterion isolated from *Angelica gigas* root (candidate). (a): before treatment with the candidate; (b): 30 min treatment with the candidate; and (c): 1 hr treatment with the candidate.
FIG. 40 shows the change in size or shape or the restoration of mobility of mutant luterial when it is treated with luterion isolated from kiwifruit (candidate). (a): before treatment with the candidate; (b): 30 min treatment with the candidate; and (c): 1 hr treatment with the candidate.
FIG. 41 shows the results of analyzing the inhibition of proliferation of AsPC-1 (pancreatic cancer cell line) when the cancer cell line was treated with *Rhus verniciflua* stokes-derived luterion (a), *Forsythiae fructus*-derived luterion (b), *Poria* cocas-derived luterion (c), *Angelica gigas* root-derived luterion (d), and kiwifruit-derived luterion (e).
FIG. 42 shows the results of analyzing the inhibition of proliferation of A549 (lung cancer cell line) when the cancer cell line was treated with *Rhus verniciflua stokes*-derived luterion (a), *Forsythiae fructus*-derived luterion (b), *Poria cocas*-derived luterion (c), *Angelica gigas* root-derived luterion (d), and kiwifruit-derived luterion (e).
FIG. 43 shows the results of analyzing the inhibition of proliferation of BT-20 (breast cancer cell line) when the cancer cell line is treated with *Rhus verniciflua stokes-*derived luterion (a), *Forsythiae fructus*-derived luterion (b), *Poria cocas*-derived luterion (c), *Angelica gigas* root-derived luterion (d), and kiwifruit-derived luterion (e).

### BEST MODE FOR CARRYING OUT THE INVENTION

Unless defined otherwise, all technical and scientific terms used herein have the same meaning as commonly understood by skilled experts in technical fields to which the invention pertains. Generally, the nomenclature used herein and the experiment methods, which will be described below, are those well known and commonly employed in the field.

As used herein, the terms "luterial" and "luterion" named by the present inventors refer to a living organism or particle present in animals and plants, respectively, and fine substances having a size ranging from a size similar to that of virus to about 500 nm (50-500 nm (normal fission stage)/800 nm or more (abnormal fusion stage)).

Luterial and luterion, which are used in the present invention, are discriminated from exosomes or microvesicles in that they include DNA and RNA and are mobile and adherent. It is known that mitochondria are colored by Janus green B and fluorescent dyes, including Rhodamine 123, Mitotracker, Acridine Orange, and DAPI, and it was found that luterion is also colored by the same dyes as those for mitochondria. Like mitochondria, luterion which has a multiple ring-like membranes without internal cristae structure, and is observed in the same laser wavelength range as that for mitochondria. In this respect, luterion may also be referred to as "pseudo-mitochondria", "mitochondria analog" or "proto-mitochondria".

In the case of mammals (including humans), a substance which is referred to as "luterial" is present in blood, saliva, lymphatic ducts, semen, vaginal fluids, mother's milk (particularly colostrums), umbilical cord blood, brain cells, red blood cells, stem cells, spinal cords, and marrow. In addition, a substance which is referred to as "luterion" is present in plants. In the case of the plants, luterion is present in a large amount in the stem parts.

Normal luterials and luterions have a size of 50-200 nm, and mutant luterials and luterions formed by fusion have a size of a few tens of micrometers. The term "luterial" and "luterion" may refer to proto mitochondria containing mRNA, miRNA and DNA. Luterial and luterion are unique in that it does not dissolve in digestive fluid and infiltrates into blood. It is expected that luterial and luterion will be associated with not only signal transduction, cell differentiation and cell death, but also the regulation of cell cycling and cell growth. The present inventors have found that luterial is closely associated with the diagnosis of cancer (PCT/KR2014/00393).

Normal luterial and luterion are expected to function to prevent the growth of cancer cells and return cells to a healthy immune state, and the functions thereof are presumed to be performed by its RNAi (RNA interference) activity that works to normalize genes.

Among the normal luterial and luterion, luterial having a size of 200 nm or less, for example, 50-150 nm is expected to function to prevent the growth of cancer cells and return cells to a healthy immune system, and the function thereof is performed by RNAi (RNA interference) having a potential to normalize genes. When an information system in RNA in the blood of healthy people or animals deviates from a normal state and directs to produce a protein that causes an abnormal disease, normal luterial will deliberately interfere with the information system so as to inhibit the development of diseases such as cancer. When luterial grows to a size of 200-500 nm or more, it will also be involved in energy metabolism, and when luterial is irradiated with light having a certain wavelength, it will function to amplify light energy in response and will act like chlorophyll. Thus, if luterial does not perform normal functions, it can cause a serious disorder in homeostasis and ATP production and can cause diseases in both respiration and energy metabolism.

It was found that luterial has the following characteristics:
(a) in normal state; it has a size (50-200 nm) smaller than that of erythrocyte, is circular or oval in shape, and is mobile
(b) it contains nucleic acids;
(c) it shows a reaction similar to that of mitochondria in fluorescence staining test;
(d) it undergoes fusion and/or fission;
(e) it grows to a size of 300 nm in the absence of fusion, grows to DNA-containing pseudo-mitochondria, and shows a structure similar to that of mitochondria in SEM or TEM images;
(f) it grows to a size of several thousands of nm in the presence of fusion;
(g) the patient-derived luterial has a size (major diameter: 500 nm or more) greater than that of healthy person-derived luterial and is mutated to form mutant luterial having a non-uniform morphology; and
(h) it shows a light reaction different from that of exosomes.

Additionally, luterial may have one or more of the following characteristics:
(i) it is autofluorescent;
(j) it produces ATP in a size of 200-400 nm;
(k) it is adherent;
(l) it has a multiple ring-like membrane structure;
(m) mutant luterial bursts in a certain environment and has stemness after bursting;
(n) it has a function of regulating p53 gene and telomeres; and
(o) it has surface antigens of CD39 or CD73.

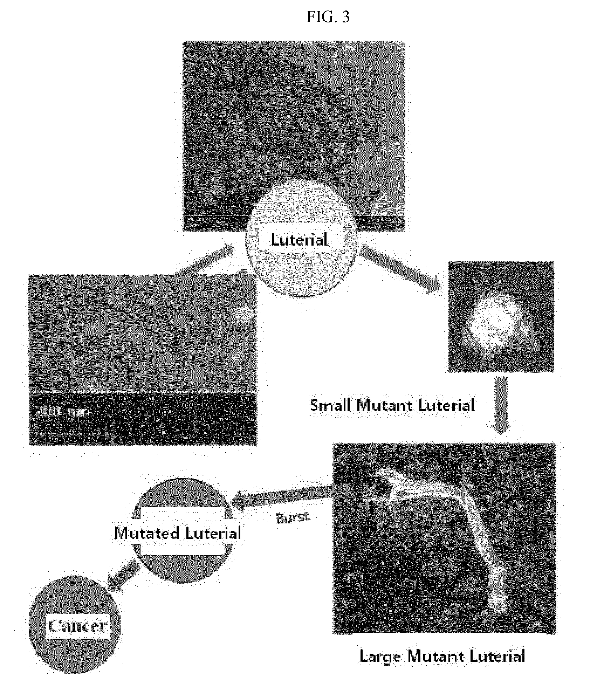

On the contrary, cancer patient-derived luterials that cannot perform normal functions as described above show phenomena and characteristics different from those of normal luterials and have various sizes or shapes (FIGS. 2 and 3). Specifically, normal luterials ceases to grow after they form double spores, but mutant luterials that are found in body fluids discharged from cancer patients or patients with chronic diseases have the property of growing infinitely, similar to stem cells, and thus have a size ranging from 800 nm to 200 µm (200,000 nm) or even bigger (PCT/KR2014/00393). In order to distinguish such luterial from normal luterial, such luterial is referred to as "mutated luterial", "luterial mutant", or "mutant luterial".

Meanwhile, the luterial isolated from humans or animals has a difficulty in observing because it quickly disintegrates or changes shape e *in vitro.* Furthermore even the normal luterial is morphologically changed into mutant luterial within 24 hours under an abnormal environment, making it harder to accurately diagnose or treat diseases.

However, luterion derived from plants has the properties of not disintegrating quickly even at room temperature, unlike the blood-derived luterial, as well asit is not mutated by fusion even though it is stored for a long period of time. In addition, the luterion can react with the mutant luterial derived from the blood of patients in order to inhibit the growth of luterial resulting in blocking the fusion of luterial. Furthermore, the luterion can inhibit the maturation of luterial derived from the blood of patients thereby preventing luterial from being mutated or grown by fusion.

As the plant-derived luterion has the RNAi function, it is expected that the use of this function can inhibit or prevent the mutation or growth of luterial derived from a patient with a specific disease, which means that the luterion can be used as an agent for treating or preventing the specific disease.

Step (a) of the present invention is to isolate luterial from a body fluid extracted from a patient or a healthy person. "The body fluid extracted from a patient" indicates blood, saliva, lymphatic ducts, semen, vaginal fluids, mother's milk (particularly colostrums), umbilical cord blood, brain cells, red blood cells, stem cells, spinal cords, or bone marrow, but is not limited thereto.

"Cancer" herein is preferably selected from the disease group of brain cancer, head and neck cancer, breast cancer, thyroid cancer, lung cancer, stomach cancer, liver cancer, pancreatic cancer, small intestine cancer, colorectal cancer, kidney cancer, prostate cancer, uterine cervical cancer, endometrial cancer and ovarian cancer, but is not limited thereto.

In one method, the luterial can be isolated from blood of a cancer patient through the following steps.
(1) removing platelet and blood-derived substances having a size greater than that of platelet from blood; (2) centrifuging the blood fraction after the removal of the platelet and the blood-derived substances having a size greater than that of platelet; (3) isolating luterial by collecting the supernatant obtained from the centrifugation; and (4) washing the isolated luterial.

Specifically, Step (1) may comprise a step of passing the blood collected from a patient through a filter having a pore size of 0.8-1.2 µm and removing unfiltered substances. Step (2) may comprise a step of repeatedly centrifuging the blood at 1,200-5,000 rpm for 5-10 minutes to remove general microvesicles such as exosomes and recovering the supernatant. Step (3) may comprise a step of exposing the supernatant recovered from step (2) to infrared light and isolating the mobile luterial particles, which are gathered toward light, by pipetting. Luterial is auto-fluorescent and mobile, and thus the luterial particles in the supernatant can be isolated by pipetting luterial observed under a dark-field microscope or a confocal microscope after its exposure to infrared light. Luterial isolated in step (3) may be passed through a filter having a pore size of 50 nm, and an unfiltered portion may be washed out with PBS. As luterial has a major diameter of 50 nm or more, blood-derived micro-substances smaller than luterial can be removed by the procedure described above. Luterial having a major diameter of 50-800 nm can be obtained, and observed through the dark-field microscope or the confocal microscope. The obtained luterial can be divided according to its size into 50-200 nm (developmental phase)/ 200-400 nm (maturation phase)/ 400-600 nm (mitosis phase)/ 600-800 nm (over-mitosis phase)/ 800nm or more (mutant) by the sequential use of 200 nm, 400 nm, 600 nm, 800 nm, and 1000 nm filters.

In another method, particles having immobilized antibody that bind specifically to a luterial surface antigen may be added to blood to induce the binding between luterial and the particles, and luterial bound to the particles may be recovered and separated. Herein, luterial surface antigen is CD39 or CD73, and luterial can be obtained by adding anti-CD39 or anti-CD73 antibody immobilized to magnetic particles, and separating only the luterial-bound particles by using a magnet (application of magnetism), and then recovering luterial (Korean Patent Application No. 10-2015-0004287).

Normal luterials in healthy persons merely form double spores (fission), but luterials (mutant luterials) in patients with chronic disease or cancer have the properties of fusing, coagulating with one another or bursting to adhere to cells such as erythrocytes or cancer cells, thereby increasing their size abnormally. Mutant luterials are highly adherent, thus the fusion is accelerated by the above-described cycle, thereby increasing their size to about 800 nm or more in major diameter and/or minor diameter, and any of such mutant luterials may also have a size of 200 µm (200,000 nm) or more.

Step (b) is a step of treating either the mutant luterial isolated from the body fluid extracted from the patient, or luterial extracted from the healthy person, with an anticancer agent or cancer preventive candidate.

In one example, the candidate may be a collection of agents that maintain the normal state of luterial or recover the abnormal state of luterial to the normal state. The candidate may be one or more selected from the group consisting of natural extracts, food- or plant-derived luterions, RNAi, apatamers, and compounds, but is not limited thereto.

The candidate may be, for example, an allergen-removed *Rhus Verniciflua* Strokes extract or a combination of an allergen-removed *Rhus Verniciflua* Strokes extract and various additional food or medicinal plant extracts. Specifically, the candidate may be a luterion-containing extract from allergen-removed *Rhus Verniciflua* Strokes.

The allergen-removed *Rhus Verniciflua stokes* extract can be obtained by harvesting the bark of *Rhus Verniciflua stokes,* and heating the collected *Rhus Verniciflua stokes* at a temperature of 25 to 100°C, at a pressure that is 0.01-1 atm higher than the atmospheric pressure, and at an oxygen concentration of 25-100% (v/v) in order to prevent allergy by the toxicity of the *Rhus Verniciflua stokes.*

In addition to the allergen-removed *Rhus Verniciflua stokes* extract, other food or medicinal plant extracts or fractions thereof may be obtained according to a known method, and mixed with the allergen-removed *Rhus Verniciflua stokes* extract at the same ratio.

In one example, the candidate may be selected from among RNAi (miRNA or siRNA), food- or plant-derived luterions and aptamers. Specifically, it may be an RNAi molecule against characteristic miRNA that is expressed in cancer cells. The food- or plant-derived luterion is an RNAi molecule that may be contained in luterion having a major diameter and/or minor diameter of 50-500 nm to exhibit RNA interference. For example, the food- or plant-derived luterion may be an RNAi molecule contained in luterion derived from foods or medicinal plants such as *Rhus verniciflua stokes.*

The miRNA, a 21-25-nucleotide single-stranded non-coding RNA molecule, controls the expressions of genes in eukaryote. The miRNA is known to bind to the mRNA 3' untranslated region (UTR) of a certain gene to inhibit the translation of the gene. Actually, miRNAs studied in animals reduce the expression of proteins without influencing the mRNA level of a certain gene. The miRNA is linked to RISC (RNA-induced silencing complex) to bind complementarily to a certain mRNA, but the central region of the miRNA remains mismatched, therefore the miRNA does not degrade mRNA, unlike conventional siRNA. Unlike animal miRNAs, food or plant miRNAs completely match their target mRNA to induce mRNA degradation, thereby inducing RNA interference.

The siRNA plays a certain role in an RNA interference (RNAi) pathway, particularly an RNA silencing pathway that is a sequence-specific RNA degradation process that is stimulated by double-stranded RNA (dsRNA). The siRNA is derived from a longer dsRNA precursor that has a small 3'-overhang which induces silencing. It acts as a guide that directs degradation of a target RNA.

The term "food- or medicinal plant-derived luterion" means one isolated from a luterion-containing food or plant extract using a method similar to that for luterial isolated from blood. The term "luterion" named by the present inventors refers to a living organism present in plants or foods meaning a fine substance with a size ranging from a size similar to that of virus to about 500 nm (50-500 nm (normal fission stage)/800 nm or more (abnormal fusion stage)).

The luterion is distinguished from exosomes or microvesicles as it contains DNA and RNA and is mobile and adherent. It is known that mitochondria are positively stained by Janus green B and fluorescent dyes, including Rhodamine 123, Mitotracker, Acridine Orange, and DAPI. The luterion is also positively stained by the same dyes as those for mitochondria. The luterion has a multiple ring-like membrane structure but has no internal cristae structure. It is observed in the same laser wavelength range as that for mitochondria. In this respect, the luterion may also be referred to as "pseudo-mitochondria", "mitochondria analog" or "proto-mitochondria".

Food- or plant-derived luterion does not dissolve or disappear within a short time even at room temperature, unlike blood-derived luterial, and is not mutated by fusion even though it is stored for a long period of time. In addition, the luterion can react with mutant luterial derived from the blood of patients to inhibit the growth of luterial so that fusion of luterial will no longer occur. Furthermore, the luterion can inhibit the maturation of luterial derived from the blood of patients thereby preventing luterial from being mutated or grown by fusion.

It is believed that the inhibitory functions of the food- or plant-derived luterion are attributable to the RNAi function of the luterion. Thus, it is expected that the use of this RNAi function can inhibit or prevent the mutation or growth of luterial derived from a patient with a specific disease, indicating that the luterion can be used as an agent for treating or preventing the specific disease.

The food- or plant-derived luterion has a density of 1 or lower, which is higher than those of fats and lipids, and lower than those of proteins. Thus, it can be isolated from plants by a steam distillation process, but is not limited thereto.

For example, the food- or plant-derived luterion may be isolated by a method comprising the steps of:
(a) adding a solvent to a food or a plant, and shaking the solution with intermittent bubbling using air or oxygen at a temperature of 50 to 90°C; (b) capturing steam or gas, which is generated by the shaking, and cooling the captured steam or gas to obtain a condensate; (c) filtering the condensate through a filter having a pore size of 0.8-1.2 µm; (d) centrifuging the condensate; and (e) isolating the food- or plant-derived luterion from the centrifuged supernatant (Korean Patent Application No. 10-2015-00001195).

In some cases, particles having immobilized antibody that binds specifically to a luterial surface antigen may be added to the luterion-containing food or plant extract to induce the binding between luterial and the particles. The luterial bound to the particles may be recovered and separated. Herein, luterial surface antigen is CD39 or CD73, and luterial can be obtained by immobilizing anti-CD39 or anti-CD73 antibody, which binds specifically to each antigen, onto magnetic particles, and separating only luterial-bound particles by use of a magnet (application of magnetism), and then recovering luterial(Korean Patent Application No. 10-2015-0004288).

Through the above steps, motile luterions having a major diameter and/or minor diameter of 50-80 nm responding to IR light can be isolated. The mobility of the luterion can be observed by a dark-field microscope or a confocal microscope.

The food- or plant-derived luterion obtained by the above procedure can be observed through the dark-field microscope or the confocal microscope. The obtained luterion can be divided according to size into 50-200 nm (developmental phase)/ 200-400 nm (maturation phase)/ 400-600 nm (mitosis phase)/ 600-800 nm (over-mitosis phase) by the sequential use of 200 nm, 400 nm, 600 nm, and 800 nm filters.

The food- or plant-derived luterion isolated by the above method has the following characteristics:
(a) it has a major diameter and/or a minor diameter of 50-800 nm, is circular or oval in shape, and is mobile;
(b) it contains nucleic acids;
(c) it shows a reaction similar to that of mitochondria in fluorescence staining;
(d) it undergoes fusion and/or fission events;
(e) it grows to a size of 500 nm in the absence of fusion, grows to DNA-containing pseudo-mitochondria, and shows a structure similar to that of mitochondria in SEM or TEM images;
(f) it shows a light reaction contrary to that of exosomes; and
(g) it generates fission during the irradiation of IR.

In some cases, the luterion may have one or more of the following characteristics:
(i) it is auto-fluorescent;
(j) it produces ATP in a size of a major diameter and/or a minor diameter of 200-400 nm;
(k) it is adherent;
(1) it inhibits fusion of blood-derived luterial or promote fission at the time of reaction with the blood-derived luterial; and
(m) it has surface antigens of CD39 or CD73.

The food- or plant-derived luterions may be classified into the following four kinds and stored: 50-200 nm; 200-400 nm; 400-600 nm; and 600-800 nm. After isolation of luterion, careful attention is required as the luterion can be dissolved or deformed during long term storage. When the luterion is to be stored for a long period of time, it is preferably cooled to -80°C or below within a short time and stored. When the luterion is to be stored in a live state for a short period of time, it is preferably stored in 0.5% saline at about 4°C while it is exposed to low-temperature IR at a distance of 30 cm. When it is to be stored for a short period of time, it may be stored in PBS solution and may also be stored under nitrogen. In some cases, the food- or plant-derived luterion may be stored in the presence of one or more preservatives selected from flavonoid fisetin, butein, and sulfuretin.

In some cases, plant-derived luterion may be cultured in a liquid at 18 to 30°C while it is exposed to IR light. Alternatively, plant-derived luterion having a major diameter and/or minor diameter of 400-800 nm may be cultured in a liquid at 18 to 30°C (preferably 20 to 25°C) while it is exposed to IR light, thereby inducing fission of the plant-derived luterion. The liquid that is used in the incubation process may be saline or PBS, but is not limited thereto. The plant-derived luterion before culturing may be obtained according to the above-described isolation method, and may have a major diameter and/or minor diameter of 50-500 nm. The plant-derived luterion cultured according to the culturing method of the present invention may have a major diameter and/or minor diameter of 300-500 nm after culture. While in culture, the size of the luterion can be controlled to have a major diameter and/or minor diameter of 500 nm or less under observation with a microscope. After completion of culture, the luterion may be classified according to the size, and cooled and stored at -80°C. Alternatively, it may also be stored under nitrogen or stored at a temperature above zero. For storage, preservative may be added to the luterion.

Plants are not limited, because luterion is present in all plants. Preferably, a medicinal plant selected from among those shown in Tables 1 to 4 may be used in the present invention. Because it is believed that luterion is distributed abundantly in the stem of plants, it is preferable to isolate luterion from the stem portion.

**Table 1**

| Group | Medicinal Plant (Botanical) Names |
|---|---|
| | *Ostericum koreanum* Maximowicz |
| | *Aralia* continentalis Kitagawa |
| | *Schizonepeta tenuifolia* Briquet |
| | *Saposhnikovia divaricata* Schischkin |
| | *Rehmannia glutinosa* Liboschitz ex Steudel |
| | *Poria cocos* Wolf |
| | *Angelica decursiva* Franchet et Savatier |
| | *Plantago asiatica* Linne |
| | *Bupleurum falcatum* Linne |
| | *Alisma orientale* Juzepzuk |
| | *Akebia quinata* Decaisne |
| A | *Scrophularia ningpoensis* Hemsley |
| | *Trichosanthes kirilowii* Maximowicz |
| | Polyporus umbellatus Fries |
| | *Coptis japonica* Makino |
| | *Sophora flavescens* Solander ex Aiton |
| | *Phellodendron amurense* Ruprecht |
| | *Anemarrhena asphodeloides* Bunge |
| | *Rehnannia glutinosa* Liboschitz ex Steudel |
| | *Cornus officinalis* Siebold et Zuccarini |
| | *Paeonia fuffruticosa* Andrews |
| | *Rubus coreanus* Miquel |
| | *Lonicera japonica* Thunberg |
| | *Mentha arvensis* Linne var. *piperascens* Malinvaud ex Holmes |
| | *Gardenia jasminoides* Ellis |
| | *Forsythia viridissima* Lindley |
| | *Arctium lappa* Linne |

**Table 2**

| Group | Medicinal Plant (Botanical) Names |
|---|---|
| | *Actinidia arguta* PLANCH |
| | *Actinidia arguta* Fructus |
| | *Chaenomelis* Fructus |
| | *Vitis vinifera* Radix |
| | *Phragmitis* Rhizoma |
| C | *Prunus tomentosa* Thunb |
| | *Acanthopanax sessiliflorum* SEEM |
| | *Pinus densiflora S. et Z* |
| | Rice bran on a mallet head |
| | *Pinus tabulaeformis* |
| | *Semen Fagopyri* |
| | *Rhus verniciflua* |

**Table 3**

| Group | Medicinal Plant (Botanical) Names |
|---|---|
| | *Rhus verniciflua* |
| | *Cnidium officinale* Makino |
| | *Angelica Gigas* Nakai |
| | *Citri Unshius* Pericarpium |
| | *Polygonum multiflorum* Thunberg |
| | *Cynanchum wilfordii* Hemsley |
| | *Panax ginseng* C. A. Meyer |
| | *Atractylodes japonica* Koidzumi |
| | *Atractylodes lancea* De Candlle |
| | *Zingiber officinale* Roscoe |
| | *Cinnamomum cassia* Presl |
| | *Citrus unshiu* Markovich |
| | *Agastache rugosa* O. Kuntze |
| | *Perilla frutescens* Britton var. *acuta* Kudo |
| U | *Zizyphus jujube* Miller var. *inermis* Rehder |
| | *Glycyrrhiza uralensis* Fischer |
| | *Aconitum carmichaeli* Debeaux |
| | *Cyperus rotundus* Linne |
| | Astragalus membranaceus Bunge |
| | *Paeonia lactiflora* Pallas |
| | *Foeniculum vulgare* Miller |
| | *Alpinia officinarum* Hance |
| | *Areca catechu* Linne |
| | Pinellia ternata Breitenbach |
| | Arisaema amurense Maximowicz var. serratum Nakai |
| | *Alpinia oxyphylla* Miquel |
| | *Poncirus trifoliata* Rafinesque |
| | *Magnolia ovobata* Thungerg |
| | *Aucklandia lappa* Decne |
| | *Evodiae rutaecarpa* Bentham |
| | *Psoralea corylifolia* Linne |
| | *Allium fistulosum* Linne |
| | *Amomum villosum* Loureiro |
| | *Crataegus pinnatifida* Bunge |

**Table 4**

| | Medicinal Plant (Botanical) Names |
|---|---|
| | *Ephedra sinica* Staph |
| | *Chrysanthemum indicum* Linne |
| | *Platycodon grandiflorum* |
| | *Prunus armeniaca* var. ansu Max. |
| | *Angelica dahurica* BENTH. Et HOOK |
| | Liriope muscari BALL |
| | *Asparagus cochinchinensis* Merr |
| | Dioscroea japonica THUNB |
| | Zizyphus jujube |
| | *Dimocarpus longan* Lour |
| G | *Polygala tenuifolia* |
| | *Acorus graminens* SOLAND |
| | *Schizandra chinensis* BAALL |
| | *Castanea crenata* S. et Z. |
| | *Coix lachrymal-jobi* var. ma-yuen |
| | *Raphanus sativus* L |
| | *Pueraria thunbergiana* |
| | *Scutellaria baicalensis* GEORG |
| | Angelica tenuissima NAKAI |
| | *Cervi Parvum* Cornu |
| | *Rhuem palmatum* |
| | *Cimicifuga heracleifolia* KOM |
| | *Boita orientalis* ENDL |
| | *Morus alba* L |
| | *Tussilago* farfara |
| | *Gingko biloba* L |
| | *Thymus* vularis |
| | *Gleditsia japonica* Miquel var. *koraiensis* Nakai |
| | Rhus verniciflua |

Aptamers are the nucleic acid molecules that show high specific binding affinity to molecules through interactions other than traditional Watson-Crick base-pairing. The aptamer can bind specifically to a selected target to regulate the activity of the target. For example, the activity of the target can be blocked by aptamer binding. The aptamers with a size of 10-15 kDa (30-45 nucleotides) bind to their targets with a nanomolar affinity or lower, and can also discriminate between closely related targets.

The candidate that is used in the present invention can be obtained from libraries of synthetic or natural compounds. A method for obtaining a library of compounds is well documented, which is commercially available from Brandon Associates (Merrimack, NH) and Aldrich Chemical (Milwaukee, WI). Libraries of natural compounds in the form of bacterial, fungal, food, medicinal plant, and animal extracts are commercially available from a number of sources, including Biotics (Sussex, UK), Xenova(Slough, UK), Harbor Branch Oceangraphics Institute(Ft. Pierce, FIa.), and PharmaMar, U.S.A.(Cambridge, Mass.).

Step (c) is a step of selecting, as an anticancer agent, a candidate that reduces the size, changes the shape or increases the mobility of mutant luterial, compared a control without treatment with the candidate. Alternatively, step (c) is a step of selecting, as a cancer preventive agent, a candidate that suppresses an increase in the size, minimizes a change in the shape or maintains the mobility of luterial, compared to a control without treatment with the candidate.

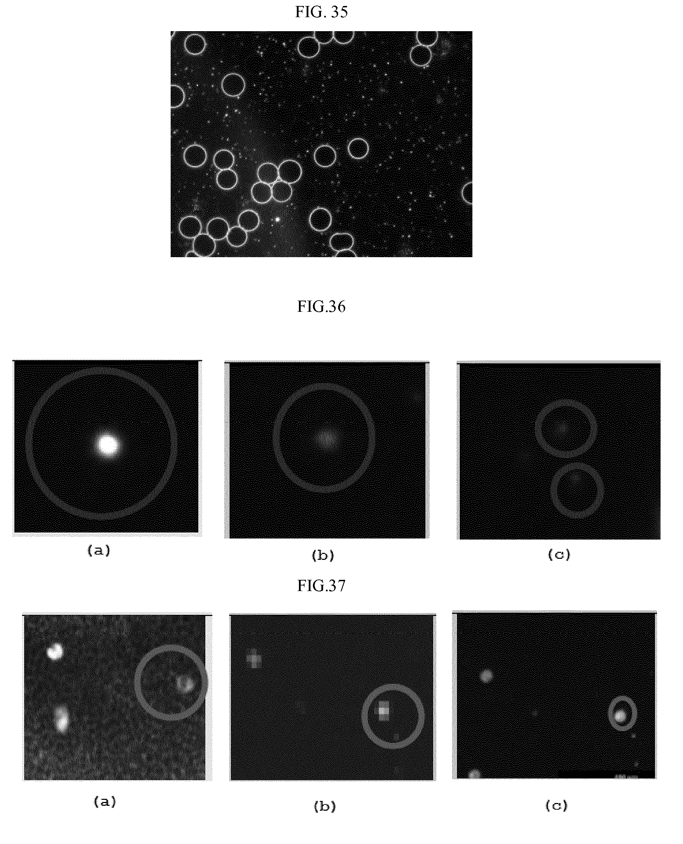

Normal luterials no longer grow after they form double spores (FIG. 35), but mutant luterials that are found in the blood of cancer patients have the property of growing infinitely, similar to stem cells, and thus have a size ranging from 800 nm or more to 200µm (200,000 nm) or more. In addition, the mobility of mutant luterial in the blood of cancer patients is significantly lower than that of normal luterial. This mobility of luterial can be quantified by measuring the nano-tracking speed of luterial.

In one example, a candidate that reduces the size of mutant luterial, compared to a control mutant luterial without treatment, may be selected as an anticancer agent. In step (c), the mutant luterial of the cancer patient has abnormal fusion in the absence of the anticancer agent candidate, throughout the formation of a deformed aggregate, causing that the size thereof is greater than that of normal luterial. However, when fusion of luterial is inhibited by the presence of the anticancer agent candidate so as to decrease the size, the candidate can be selected as an anticancer agent.

In step (c), the mutant luterial derived from the cancer patient may have a major diameter and/or minor diameter of, for example, about 1000 nm to 3500 nm (up to 200 µm) in the absence of the anticancer agent candidate. However, at 30 minutes or more (preferably 1 hour or more) after treatment with the candidate, the size of luterial may decrease to a size of 200-2,000 nm, compared to the control without treatment with the candidate.

In addition, the candidate may be selected as an effective anticancer agent when the size of the mutant luterial decreases to about 70% or less of the diameter of the control mutant luterial measured before treatment with the candidate. For example, when the mutant luterial decreases tot 10-70% of the major diameter and/or minor diameter of the control mutant luterial after a 30 minute (preferably 1 hour or more) treatment with the candidate, the candidate may be selected as an effective anticancer agent. In Test Example 1 of the present invention, it was found that, after 1 hour treatment with luterion derived from *Rhus verniciflua stokes, Forsythiae fructus, Poria cocas, Angelica gigas* root and kiwifruit, which are anticancer agent candidates, the major diameter of mutant luterial in each treated group decreased to 13-63% of the major diameter of the control mutant luterial before treatment, and the minor diameter of the mutant luterial decreased to 12-67% of the minor diameter of the control mutant luterial before treatment..

In one example, a candidate that changes the shape of mutant luterial compared to the control before treatment may be selected as an anticancer agent.

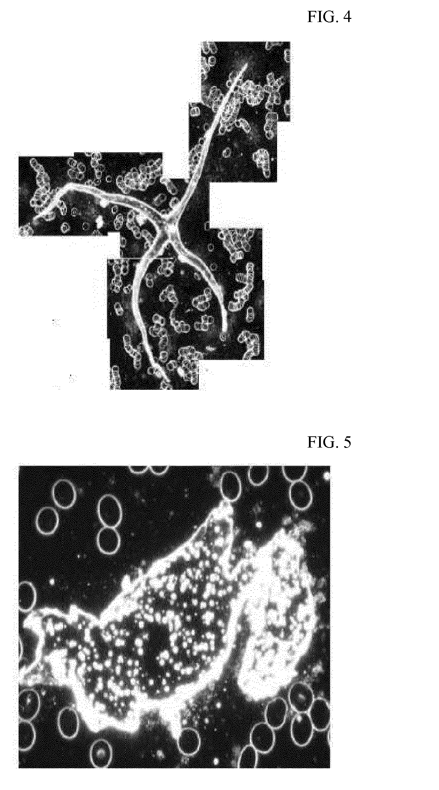

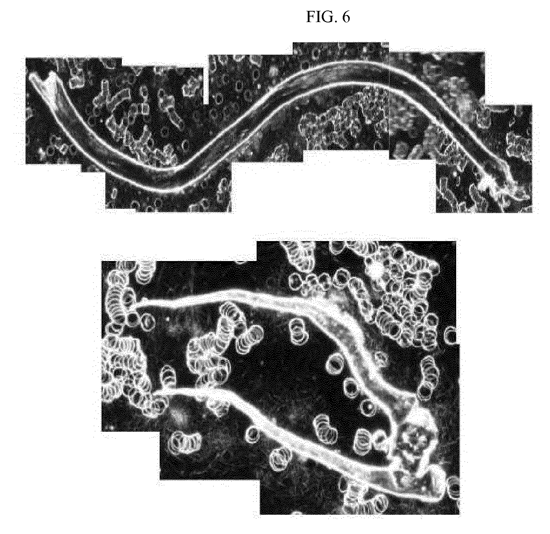

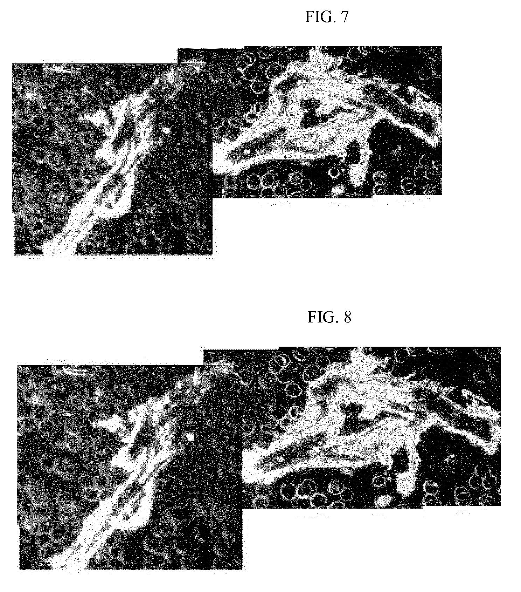

When the shape of luterial derived from a cancer patient is changed by the presence of an anticancer agent candidate, the candidate may be selected as an anticancer agent. The reduction of the mutant shape of luterial may indicate that the number of mutant luterials having a flagellum shape, a micro-tubular shape, a mass shape, a rod shape or a combination shape is being decreased, or the mutant luterials are being restored to a circular or oval shape that is observed in the luterial of healthy persons. For example, when the number of luterials having a flagellum shape (FIG. 4), a micro-tubular shape (FIG. 5), a mass shape (FIG. 6), a rod shape (FIG. 7) or a combination shape decreases to 80% or less, preferably 50% or less, more preferably 30% or less of the control mutant luterials after 30 minutes or more, or preferably 1 hour or more, with an anticancer agent candidate, that particular anticancer agent candidate may be selected as an anticancer agent. When the number of mutant luterials having a flagellum shape, a micro-tubular shape, a mass shape, a rod shape or a combination shape decreases to 5-80%, preferably 10-50%, more preferably 10-30%, of a control (in which an anticancer agent candidate is not present) by treatment with the anticancer agent candidate, the anticancer agent candidate may be selected.

In the case of suspected malignancy with the flagellum-shaped luterial, most patients were likely to be diagnosed as stage IV cancer. Thus, a screened therapeutic agent reducing the flagellum shape or restoring it to a circular or oval shape may be a therapeutic agent suitable for treatment of stage IV cancer patients.

In the case of suspected malignancy with the mass-shaped luterial, the tumor was likely to be found in the liver, colon, digestive organs, male rectum, and/or female uterus. Thus, a screened therapeutic agent reducing the mass shape or restoring it to a circular or oval shape may be a therapeutic agent suitable for treatment of cancer of the liver, colon, digestive organs, male rectum, and/or female uterus.

In the case of suspected malignancy with the rod-shaped luterial, the tumor was likely to be found in the lung, pancreas, thyroid, bone, brain, male prostate, female ovary and/or breast. Thus, a screened therapeutic agent reducing the rod shape or restoring the shape to a circular or oval shape may be a therapeutic agent suitable for treatment of cancer of the lung, pancreas, thyroid, bone, brain, male prostate, female ovary and/or breast.

In the case of suspected malignancy with luterial having a combination shape consisting of the mass shape and the rod shape, the patients were likely to be diagnosed to have stage IV metastatic cancer. Thus, a screened therapeutic agent reducing the combination shape or restoring the shape to a circular or oval shape may be suitable for the prevention or treatment of stage IV metastatic cancer.

In another example, the mobility of mutant luterial may decrease, with a nano-tracking speed ranging from less than 10 nm/sec to 0.5 nm/sec or 0 nm/sec (no mobility). However, when the degree of decrease in the mobility is reduced or reserved after a 30 minute or more (preferably 1 hour or more) treatment with a candidate, or when the nano-tracking speed is restored to 12 nm/sec or more, for example, 50-600 nm/sec, preferably 100-500 nm/sec, after treatment with the candidate, the candidate may be selected as an effective anticancer agent.

The present invention claims that the change in mobility by treatment with a candidate can be determined by measuring electrophoretic mobility. As used herein, the term "electrophoretic mobility" refers to a value obtained by dividing the speed of electrophoretic movement of charged particles by the strength of an electric field in that place. If the electrophoretic mobility of the particles is high, the mobility of the particles can also be high.

The mobility of mutant luterial may decrease, and thus the electrophoretic mobility thereof may range from less than 0.5 µm cm/Vs to 0 µm cm/Vs (no mobility). When the mobility of mutant luterial is restored after a 30 minute (preferably 1 hour or more) treatment with a candidate, and thus the electrophoretic mobility thereof increases 30% or more, for example, 30-300%, compared to a mutant luterial control before treatment with the candidate, the candidate may be selected as an effective anticancer agent. In Test Example 1 of the present invention, it was found that, after 1 hour treatment with luterion derived from *Rhus verniciflua stokes, Forsythiae fructus, Poria cocas, Angelica gigas* root and kiwifruit, which are anticancer agent candidates, the electrophoretic mobility of mutant luterial in each treated group increased 33-270% compared to the control.

The present inventors have screened anticancer agents by examining the changes in cancer patient-derived mutant luterials having a size of 1100-3100 nm and a very low mobility or no mobility after administration of candidates. As a result, it was found that the candidate may be selected as an anticancer agent when the diameter of mutant luterial decreases 70% or less of the diameter of the control mutant luterial without treatment, or when the nano-tracking speed of the mutant luterial is restored to 100 nm/sec or more, and the electrophoretic mobility increases 30% or more after one hour treatment with the candidate.

The method for screening the cancer preventive agent comprises step (c) of selecting a candidate that suppresses an increase in the size, minimizes a change in the shape, or maintains the mobility of luterial, compared to the control without treatment.

In step (c) of the method for screening the cancer preventive agent, a candidate may be selected when fusion of healthy person-derived luterials in a group treated with the cancer preventive agent candidate is not observed for 30 minutes or more, preferably 1 hour or more, indicating that the size of luterial is maintained. Also, when the major diameter or minor diameter of healthy person-derived luterial does not increase 10% or more compared to that of control luterial, the candidate may be selected as the cancer preventive agent.

In addition, a cancer preventive agent candidate may be selected when healthy person-derived luterial in a group treated with the cancer preventive agent candidate is maintained in a circular or oval shape (that is, 100% of luterial is circular or oval in shape) over 30 minutes or more, preferably 1 hour. Also, when the change of luterial shape to a flagellum shape, a micro-tubular shape, a mass shape, a rod shape or a combination shape is minimized (that is, luterial whose shape changed to a flagellum shape, a micro-tubular shape, a mass shape, a rod shape or a combination shape is 20% or less).

Moreover, a cancer preventive agent candidate may be selected as a cancer preventive agent, when the mobility of luterial in a group treated with the cancer preventive agent candidate is maintained over 30 minutes or more, preferably 1 hour or more, that is, the nano-tracking speed is maintained at 100 nm/sec or more.

In some cases, a candidate may be selected as a cancer preventive agent, by first inducing fusion of luterials (negative control) with chemical or physical treatment and second observing to see if the candidate reduces the change in size, morphology or mobility of the induced mutant luterials. In other cases, a candidate may be chosen as a cancer preventive agent, by first inducing mutation of luterials under the conditions of a temperature at 36°C and humidity at 50% or more and second observing to see if the candidate reduces the change in the size, shape or mobility of the induced mutant luterials. For example, a cancer preventive agent candidate may be selected when the increase in size, the change in shape or the decrease in mobility of luterial is reduced or inhibited after luterial is treated with a cell fusion inducer such as lysolecithin or polyethylen glycol 6000, and then incubated in the presence of the cancer preventive agent candidate.

The effects of the candidate on the size, shape or mobility of luterial can be confirmed by staining luterial with one or more dyes selected from the group consisting of Rhodamine 123, Mito-tracker, Acridine Orange, DAPI and Janus green B.

The microscope that can be used to confirm the inhibition of fusion or the change in size, shape or mobility of luterial using the dyes described above is not specifically limited as long as it is a microscope capable of observing the positive staining of luterial. Specifically, the microscope may include a dark-field microscope (Ultra microscope), a Raman spectrometer (using a wavelength of 532 nm), Leica, AFM (Atomic Force Microscope), MFM (Magnetic force microscope), STM (Scanning tunneling microscope), CLSM (Confocal Laser Scanning Microscope), NSOM (Near-field scanning optical microscope), SEM (Scanning Electron Microscope), or TEM (Transmission Electron Microscope), which is generally used by those skilled experts in the field.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Isolation of Luterial

As shown in Table 5 below, luterials were isolated from the blood of (1) lung cancer patients, (2) pancreatic cancer patients, (3) colorectal cancer patients, (4) liver cancer patients, (5) prostate cancer patients, (6) breast cancer patients, (7) thyroid papillary carcinoma patients, (8) renal cancer patients, (9) leukemia patients, (10) patients with terminal cancer (gastric cancer, colorectal cancer, gallbladder cancer), and (11) patients confirmed to have stage 4 metastatic cancer (lung cancer, prostate cancer, breast cancer). Blood was collected from patients confirmed to have cancer, and then centrifuged to settle materials in the blood. The spun blood was allowed to stand for 5-10 minutes, and then the supernatant was collected by pipetting. Then, 5 µl of CD39 antibody-conjugated iron magnetic nanoparticles or CD73 antibody- conjugated iron magnetic nanoparticles were added to 100-200 µl of the blood and incubated for 30 minutes, after which the mixture was maintained in a magnetic separator for 1-2 minutes to collect luterial bound magnetic nanoparticles, and the supernatant was removed, followed by washing. Next, 0.033 wt% BSA (Bovine Serum Albumin)/PBS buffer was added to luterial-bound iron magnetic nanoparticles, followed by incubation at 25°C for 1 hour. Then, only BSA-adsorbed iron magnetic nanoparticles were isolated using a magnet. Then, a certain amount of PBS was added to the BSA-adsorbed iron magnetic nanoparticles, followed by incubation to separate luterial by desorption. The separated luterial was quantitatively analyzed by an FP-640 spectroflurorometer (JASCO) using a standard calibration method at 280 nm (emission slit: 0.5 nm, absorption slit: 0.5 nm). Using a confocal laser scanning microscope, cancer patient-derived mutant luterial having a size of 1,000-3,200 nm was collected.

**Table 5**

| Type of cancer | Diagnosis | Shape of Luterial |
|---|---|---|
| Lung cancer | Lung cancer, metastasized to bone | Rod 2 shape (FIG. 8) |
| | Lung cancer, stage 4 squamous carcinoma, mass, T4N2M1 | cellCombination shape (FIG. 9) |
| | Lung cancer, stage 3b, T4N3M0 Lung cancer, adenocarcinoma | Rod shape (FIG. 10) Combination shape (FIG. 11) |
| | Non-small lung cancer, metastasized to brain, with adenocarcinoma | Rod shape (FIG. 12) |
| | Metastasized to supraclavicular lymph nodes and liver, squamous cell carcinoma | Combination shape (FIG. 13) |
| | Lung cancer, metastasized to bone | Combination shape (FIG. 14) |
| | Lung cancer, adenocarcinoma | Mass shape (FIG. 15) |
| | Lung cancer, adenocarcinoma | Mass shape (FIG. 16) |
| Pancreatic cancer | Pancreatic cancer, metastasized to lymph node (LN), invasive adenocarcinoma, pancreatic head | Rod 1 shape (FIG. 17) |
| | Pancreatic cancer, metastasized to liver, adenocarcinoma | Rod shape (FIG. 18) |
| Colorectal cancer | Colorectal cancer metastasized uterus, T4N2M1 (adenocarcinoma) | toMass shape (FIG. 19) |
| | Colorectal cancer (adenocarcinoma)Mass metastasized to liver and lung | shape (FIG. 20) |
| | Colorectal cancer metastasized to liver, lung and brain | Rod shape (FIG. 21) |
| | Colorectal cancer metastasized liver | toMass shape (FIG. 22) |
| Liver cancer | Liver cancer metastasized to lung | Mass shape (FIG. 23) |
| Angiosarcom a of liver | Angiosarcoma of liver | Mass shape (FIG. 24) |
| Gallbladder cancer | Bile duct-invaded gallbladderFlagellum cancer | shape (FIG. 25) |
| Prostate cancer | Prostate cancer (adenocarcinoma),Combination T2N1M1, metastasized to bone | shape (FIG. 26) |
| | Prostate cancer | Rod shape (FIG. 27) |
| Breast cancer | Stage 3 breast cancer | Rod shape (FIG. 28) |
| | Stage 3b breast cancer | Combination shape (FIG. 29) |
| Thyroid papillary carcinoma | Thyroid papillary carcinoma, poorly differentiated | Rod shape (FIG. 30) |
| Renal cancer 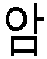 | Renal cancer | Rod shape (FIG. 31) |
| Gastric cancer | Gastric cancer, T3N1M0 | Flagellum shape (FIG. 32) |
| | Gastric cancer, adenocarcinoma | T3N3M0,Flagellum shape (FIG. 33) |
| | Stage 4 gastric metastasized to adenocarcinoma cancer, lung,Rod | shape (FIG. 34) |

### Example 2: Preparation of Candidates

100 g of the following medicinal plants were cut to fit into a container size of 2-3 liters (20- to 30-fold by volume) and placed in a container: *Rhus verniciflua stokes, Forsythiae fructus, Poria cocas, Angelica gigas* root and kiwifruit. Then 500-800 g (equivalent to 5-8 times the weight of the plant, preferably 600 g which is 6 times the weight of the plant) of distilled water was added to the container, followed by shaking at 80°C for about 8 hours, thereby obtaining a hot-water extract. CD39 antibody-conjugated iron magnetic nanoparticles or CD73 antibody-conjugated iron magnetic nanoparticles were added to 100-200 µl of the hot-water extract and incubated for 30 minutes. Next, the mixture was maintained in a magnetic separator for 1-2 minutes to collect luterion-bound magnetic nanoparticles, and the supernatant was discarded, followed by washing. Next, 0.033 wt% BSA (Bovine Serum Albumin)/PBS buffer was added to the luterion-bound magnetic nanoparticles, followed by incubation at 25°C for 1 hour. Next, only BSA-adsorbed iron magnetic nanoparticles were separated using a magnet, and a certain amount of PBS was added to the BSA-adsorbed iron magnetic nanoparticles, followed by incubation to perform desorption, thereby obtaining luterion derived from each of *Rhus verniciflua stokes, Forsythiae fructus, Poria cocas, Angelica gigas* root and kiwifruit.

Through the above-described process, luterions having a major diameter of 50-500 nm could be obtained, as could be observed by a dark-field microscope or a confocal microscope. According to the same method as described above, luterions can be obtained from the medicinal plants shown in Tables 1 to 4 below.

### Test Example 1: Anticancer Agent Screening

The mutant luterials separated in Example 1 was treated with each of the *Rhus verniciflua stokes-, Forsythiae fructus, Poria cocas-, Angelica gigas* root- and kiwifruit-derived luterion (contained in PBS buffer) obtained in Example 2. Herein, the *Forsythiae fructus-, Poria cocas, Angelica gigas* root- and kiwifruit-derived luterion were used at concentrations of 1, 5, 10, 50, 100 and 500 µg/ml (50 µg/ml corresponds to about 7 x 10⁸ luterions/ml), and the *Rhus verniciflua stokes*-derived luterion was used at concentrations of 0.1, 0.5, 1, 5, 10 and 50 µg/ml (5 µg/ml corresponds to about 7 x 10⁷ luterions/ml).

After the above treatment of mutant luterial in PBS for 30 minutes or 1 hour under the conditions of 30°C and pH 7.3, changes in the size and mobility of the mutant luterial were examined. The change in size of the mutant luterial was examined by observing the change in diameter of the mutant luterial with a confocal laser scanning microscope, and the change in mobility of the mutant luterial was examined by nano-tracking (3i Inc., USA). To examine the change in mobility, tracking was set in the center of luterial, and nano-tracking was operated. Then, the real-time movement trajectory of luterial was recorded and the speed per second of luterial was calculated, thereby measuring the nano-tracking speed. In addition, using a Malvern ZetaSizer Nano ZSP instrument, the mutant luterial was placed in the cell (Universal Dip Cell: ZEN1002), and two electrodes were immersed in the cell, after which the electrophoretic mobility was measured by observing the charged particles moving toward the electrode having the opposite charge.

As shown in Table 6 below, the size of the mutant luterial derived from the cancer patients decreased after administration of each of the *Rhus verniciflua stokes*-derived luterion (luterion size = major diameter x minor diameter: 400 nm X 350 nm), the *Forsythiae fructus*-derived luterion (400 nm X 370 nm), the *Poria cocas*-derived luterion (450 nm X 300 nm), the *Angelica gigas* root-derived luterion (400 nm X 300 nm) and the kiwifruit-derived luterion (420 nm X 330 nm), compared to those not receiving the treatment (control) The mobility of the mutant luterial was also restored after administration of each of the luterions.

**Table 6**

| Candidate | Results | Control (a) | 30-minute treatment (b) | 1-hour treatment (c) |
|---|---|---|---|---|
| *Rhus verniciflua stokes-*derived luterion | Size (major diameter X minor diameter nm) | 2100 X 2000 | 1400 X 1200 | 600 X 450 |
| | Nano-tracking speed (nm/sec) | <10 | 10-30 | 250-350 |
| | Electrophoreti c mobility (µm cm/Vs) | <0.5 | 0.65 | 0.73 |
| *Forsythiae fructus-*derived luterion | Size (nm) | 1100 X 1000 | 800 X 850 | 700 X 670 |
| | Nano-tracking speed (nm/sec) | <5 | 10-20 | 200-300 |
| | Electrophoreti c mobility (µm cm/Vs) | <0.3 | 0.38 | 0.4 |
| *Poria cocas-*derived luterion | Size (nm) | 2800 X 2100 | 2500 X 1800 | 1600 X 800 |
| | Nano-tracking speed (nm/sec) | 0 | 10 | 100 |
| | Electrophoreti c mobility (µm cm/Vs) | <0.1 | 0.22 | 0.31 |
| *Angelica gigas* root-derived luterion | Size (nm) | 3100 X 2600 | 800 X 600 | 400 X 300 |
| | Nano-tracking speed (nm/sec) | 0 | 10 | 100-200 |
| | Electrophoreti c mobility (µm cm/Vs) | <0.1 | 0.25 | 0.37 |
| Kiwifruit-derived luterion | Size (nm) | 1300 X 1100 | 600 X 450 | 350 X 330 |
| | Nano-tracking speed (nm/sec) | <10 | 10-30 | 300-500 |
| | Electrophoreti c mobility (µm cm/Vs) | <0.5 | 0.82 | 0.86 |

The results are shown in FIGS. 36 to 40. As shown in FIGS. 36 to 40, the cancer patient-derived mutant luterial control ((a) in FIGS. 36 to 40), not treated with luterions showed i) a size of about 1,100-3,100 nm, and ii) a nano-tracking speed of about 0-10 nm/sec, and an electrophoretic mobility of about 0-0.5 µm cm/Vs.

However, in the group treated with each of the *Rhus verniciflua stokes-,* the *Forsythiae fructus-,* the *Poria cocas-,* the *Angelica gigas* root- and the kiwifruit-derived luterion (after 30 min: (b) in FIGS. 36 to 40, and after 1 hour: (b) in FIGS. 36 to 40), the size of luterial significantly decreased, and the size of a portion of luterial decreased to that of normal luterial (about 800 nm or less).

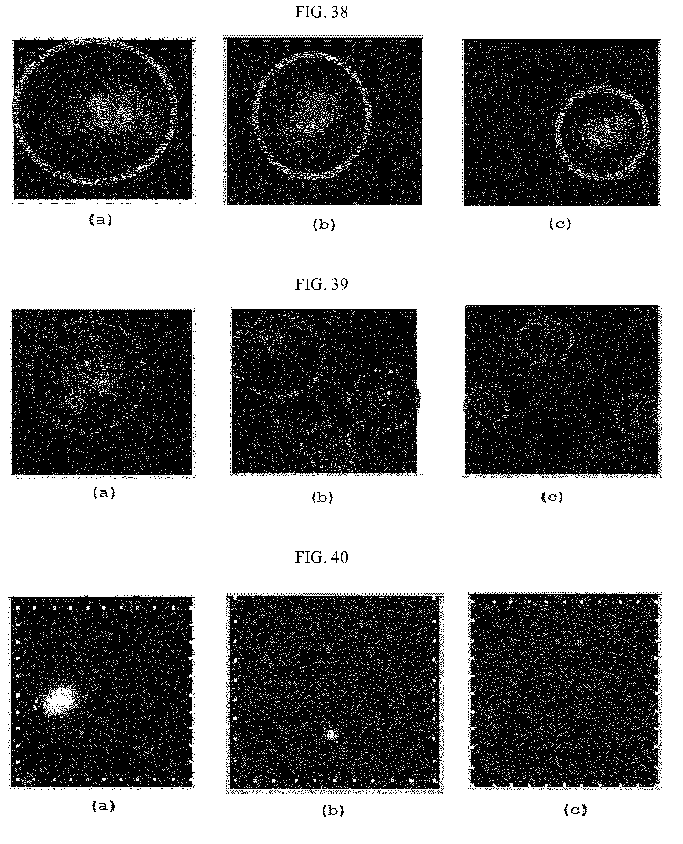

In the group treated with the *Rhus verniciflua stokes-*derived luterion (FIG. 36(c)), the size of the mutant luterial decreased to about 28% of the major diameter of the mutant luterial (control) after 1 hour, and decreased to about 22% of the minor diameter of the mutant luterial after 1 hour. In the group treated with the *Forsythiae fructus-*derived luterion (FIG. 37(c)), the size of the mutant luterial decreased to about 63% of the major diameter of the mutant luterial (control) after 1 hour, and decreased to about 67% of the minor diameter of the mutant luterial after 1 hour. In the group treated with the *Poria* cocas-derived luterion (FIG. 38(c)), the size of the mutant luterial decreased to about 57% of the major diameter of the mutant luterial (control) after 1 hour, and decreased to about 38% of the minor diameter of the mutant luterial after 1 hour. In the group treated with the *Angelica gigas* root-derived luterion (FIG. 39(c)), the size of the mutant luterial decreased to about 13% of the major diameter of the mutant luterial (control) after 1 hour, and decreased to about 12% of the minor diameter of the mutant luterial after 1 hour. In the group treated with the kiwifruit-derived luterion (FIG. 40(c)), the size of the mutant luterial decreased to about 27% of the major diameter of the mutant luterial (control) after 1 hour, and decreased to about 30% of the minor diameter of the mutant luterial after 1 hour.

Regarding the mobility, it was shown that the nano-tracking speed was lower than 10 nm/sec in the mutant luterial, but about 100-500 nm/sec in the group treated with each of the *Rhus verniciflua stokes-,* the *Forsythiae fructus-,* the *Poria cocas-,* the *Angelica gigas* root- and the kiwifruit-derived luterions, indicating that the mobility of the treated group was restored.

In addition, the results of measurement of the electrophoretic mobility show that an electrophoretic mobility lower than about 0-0.5 µm cm/Vs was measured in the mutant luterial, but was restored in the group treated with each of the *Rhus verniciflua stokes-,* the *Forsythiae fructus-,* the *Poria cocas-,* the *Angelica gigas* root- and the kiwifruit-derived luterions. Specifically, the electrophoretic mobility of the group treated with the *Rhus verniciflua stokes*-derived luterion was about 0.73 µm cm/Vs, which is about 46% higher than that of the mutant luterial (control), and the electrophoretic mobility of the group treated with the *Forsythiae fructus*-derived luterion was about 0.4 µm cm/Vs, which is about 33% higher than that of the mutant luterial (control). In addition, the electrophoretic mobility of the group treated with the *Poria* cocas-derived luterion was about 0.31 µm cm/Vs, which is about 210% higher than that of the mutant luterial (control), and the electrophoretic mobility of the group treated with the *Angelica gigas* root-derived luterion was about 0.37 µm cm/Vs, which is about 270% higher than that of the mutant luterial (control). Furthermore, the electrophoretic mobility of the group treated with the kiwifruit-derived luterion was 0.86 µm cm/Vs, which is about 72% higher than that of the mutant luterial (control).

In conclusion, it can be seen that treatment of mutant luterial with the luterion derived from each of *Rhus verniciflua stokes, Forsythiae fructus, Poria cocas, Angelica gigas* root and kiwifruit, which are medicinal plants, can reduce the fusion of luterial, reduce the size, and suppress the change in the shape, compared to a control mutant luterial, and can restore the decrease in luterial mobility that is found in cancer patients. Thus, the luterion derived from each of *Rhus verniciflua stokes, Forsythiae fructus, Poria cocas, Angelica gigas* root and kiwifruit, which are medicinal plants, was selected as an anticancer agent.

### Test Example 2: Measurement of the Effect of Selected Candidate on Cancer Cell Line Viability in vitro

The effect of each of the *Rhus verniciflua stokes-, Forsythiae fructus-, Poria cocas-, Angelica gigas* root- and kiwifruit-derived luterions, confirmed to have an anticancer effect in Test Example 1, on the inhibition of proliferation of cancer cell lines was examined. Each of AsPC-1 (pancreatic cancer cell line), A549 (lung cancer cell line) and BT-20 (breast cancer cell line), obtained from the Korean Cell Line Bank (KCLB) was cultured using 10% fetal bovine serum (FBS)-containing RPMI1640 and DMEM media.

Each of the cell lines was seeded in a 96-well plate at different concentrations depending on the growth rate thereof, and then cultured at 37°C for 16-24 hours, after which each cell line was treated stepwise with five concentrations of the *Rhus verniciflua stokes-, Forsythiae fructus-, Poria cocas-, Angelica gigas* root- and kiwifruit-derived luterions isolated in Example 2.

After 72 hours, 15 µℓ of MTT dye solution (Promega) was added to each well, followed by incubation at 37°C for 4 hours. Next, each well was treated with 100 µℓ of a developer solution/stop solution mixture, and incubated overnight at 37°C. 150 µl of DMSO was added to each well, and then the absorbance at 590 nm was measured using a microplate reader (Bio-Rad, USA), thereby determining the cell viability of each cell line.

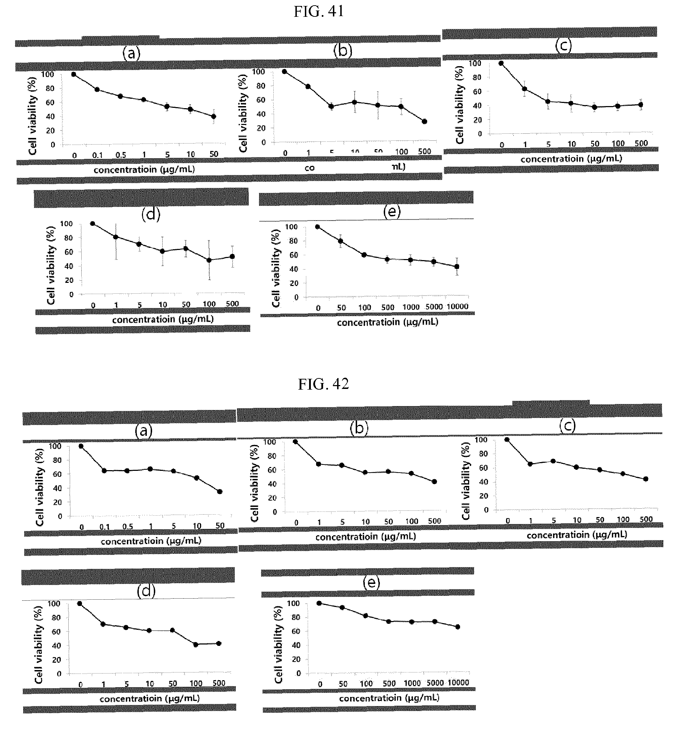

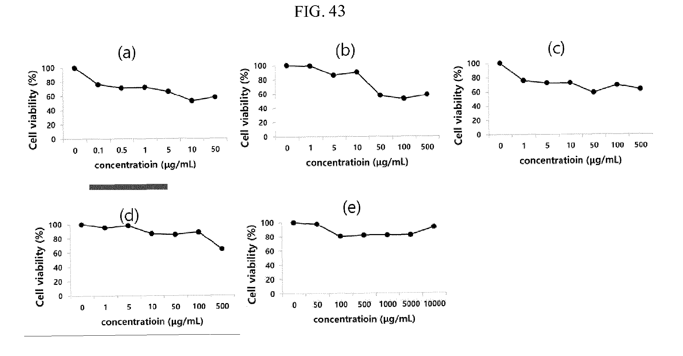

As a result, as can be seen in FIGS. 41 to 43, each of the *Rhus verniciflua stokes-, Forsythiae fructus-, Poria cocas-, Angelica gigas* root- and the kiwifruit-derived luterions reduced the viability of each of AsPC-1 (pancreatic cancer cell line), A549 (lung cancer cell line) and BT-20 (breast cancer cell line), indicating that the luterion significantly inhibited the proliferation of each of the cancer cell lines.

### INDUSTRIAL APPLICABILITY

The present invention provides the novel method capable of screening an anticancer agent or a cancer preventive agent based on mutant luterial isolated from cancer patients. According to the screening method of the present invention, an anticancer agent or a cancer preventive agent can be easily screened within a relatively short time by observing either the change in size, shape or mobility of mutant luterial, which appears when the mutant luterial is treated with an anticancer agent or cancer preventive agent candidate, or whether normal luterial is maintained in a normal state.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A method for screening an anticancer agent, comprising the steps of:
(a) isolating mutant luterial from a body fluid extracted from a patient;
(b) treating the isolated mutant luterial with anticancer agent candidates; and
(c) selecting, a candidate as the anticancer agent by testing its ability to reduce the size, change the shape or increase the mobility of the mutant luterial compared to a control mutant luterial without treatment.

2. A method for screening a cancer preventive agent, comprising the steps of:
(a) isolating normal luterial from a body fluid extracted from a normal person;
(b) culturing the isolated normal luterial in the presence of cancer preventive agent candidates; and
(c) selecting, as the cancer preventive agent, a candidate that suppresses the increase in size, minimizes the change in shape or maintains the mobility of the luterial compared to a control luterial without treatment.

3. The method of claim 1 or 2, wherein the extracted body fluid in step (a) is selected from the group consisting of blood, saliva, lymphatic ducts, semen, vaginal fluids, mother's milk, colostrums, umbilical cord blood, brain cells, spinal cords, and marrow.

4. The method of claim 1 or 2, wherein the candidate in step (b) is one or more selected from the group consisting of natural extracts, food- or plant-derived luterions, RNAi, aptamers and compounds.

5. The method of claim 4, wherein the plant-derived luterions are one or more medicinal plants selected from the group consisting of *Rhus verniciflua stokes, Forsythiae fructus, Poria cocas, Angelica gigas* root and kiwifruit.

6. The method of claim 4, wherein the plant-derived luterions have a major diameter or a minor diameter of 50-500 nm.

7. The method of claim 1, wherein step (c) comprises selecting as the anticancer agent from the candidates when the number of mutant luterials having a flagellum shape, a micro-tubular shape, a mass shape, a rod shape or a combination shape decreases to 80% or less or when the mutant luterials are restored to a circular or oval shape, compared to a control mutant luterial without treatment with the candidate.

8. The method of claim 1, wherein step (c) comprises selecting as the anticancer agent from the candidates when the size of the mutant luterial decreases to about 70% or less of the diameter of the control mutant luterial without treatment, upon 1 hour treatment with the candidate.

9. The method of claim 8, wherein the candidate is selected as the anticancer agent when the size of the mutant luterial decreases to about 70% or less of a major diameter and a minor diameter of the control mutant luterial without treatment, upon 1 hour treatment with the candidate.

10. The method of claim 1, wherein step (c) comprises selecting as the anticancer agent from the candidates when the nano-tracking speed is restored to 12 nm/sec or more compared to the control mutant luterial without treatment, upon treatment with the candidate.

11. The method of claim 10, wherein the candidate is selected as the anticancer agent when the nano-tracking speed is restored to 100-120 nm/sec, compared to that of a control mutant luterial upon treatment with the candidate.

12. The method of claim 1, wherein step (c) comprises selecting as the anticancer agent from the candidates when the electrophoretic mobility of the mutant luterial increases 30% or more, compared to that of the control mutant luterial, upon treatment with the candidate.

13. The method of claim 1 or 2, wherein step (c) comprises coloring of luterial with one or more dyes selected from the group consisting of Rhodamine 123, Mitotracker, Acridine Orange, DAPI, and Janus green B, and observing a change in the fluorescent color of luterial through a microscope.

14. The method of claim 13, wherein luterial is observed through a dark-field microscope, a Raman spectrometer, Leica, AFM (Atomic Force Microscope), MFM (Magnetic force microscope), STM (Scanning tunneling microscope), CLSM (Confocal Laser Scanning Microscope), NSOM (Near-field scanning optical microscope), SEM (Scanning Electron Microscope), or TEM (Transmission Electron Microscope).

15. The method of claim 14, wherein luterial is observed through a dark-field microscope, a Raman spectrometer, Leica, AFM (Atomic Force Microscope), MFM (Magnetic force microscope), STM (Scanning tunneling microscope), CLSM (Confocal Laser Scanning Microscope), NSOM (Near-field scanning optical microscope), SEM (Scanning Electron Microscope), or TEM (Transmission Electron Microscope).
